(19) Europäisches Patentamt
European Patent Office
Office européen des brevets

(11) EP 1 686 971 B1

(12) **EUROPEAN PATENT SPECIFICATION**

(45) Date of publication and mention of the grant of the patent:
**15.05.2013 Bulletin 2013/20**

(21) Application number: **04818410.5**

(22) Date of filing: **03.11.2004**

(51) Int Cl.:
***A61K 31/4178*** *(2006.01)* ***A61P 33/14*** *(2006.01)*

(86) International application number:
**PCT/EP2004/052763**

(87) International publication number:
**WO 2005/046656 (26.05.2005 Gazette 2005/21)**

(54) **USE OF 5-CHLORO-1-(2,6-DICHLORO-4-TRIFLUOROMETHYLPHENYL)-4-(4,5-DICYANO-1H-IMIDAZOL-2YL)-3-METHYL-1H-PYRAZOLE IN SYSTEMIC CONTROL OF ACARID INFESTATION ON ANIMALS**

VERWENDUNG VON HALOARYLPYRAZOL-VERBINDUNGEN BEI DER KONTROLLE VON PARASITENBEFALL BEI TIEREN

UTILISATION DU 5-CHLORO-1-(2,6-DICHLORO-4-TRIFLUOROMETHYLPHENYL)-4-(4,5-DICYANO-1H-IMIDAZOL-2YL)-3-METHYL-1H-PYRAZOLE POUR LE CONTRÔLE SYSTÉMIQUE DE L'INFESTATION DES ANIMAUX PAR DES ACARIENS

(84) Designated Contracting States:
**AT BE BG CH CY CZ DE DK EE ES FI FR GB GR HU IE IS IT LI LU MC NL PL PT RO SE SI SK TR**

(30) Priority: **04.11.2003 EP 03078484**

(43) Date of publication of application:
**09.08.2006 Bulletin 2006/32**

(73) Proprietor: **Intervet International BV**
**5831 AN Boxmeer (NL)**

(72) Inventors:
- **MERTENS, Christina**
**55270 Engelstadt (DE)**
- **DOHRMANN, Heike**
**55270 Sörgenloch (DE)**

(74) Representative: **Stumm, Karin et al**
**Merck Sharp & Dohme Ltd.**
**Hertford Road**
**Hoddesdon, Hertfordshire**
**EN11 9BU (GB)**

(56) References cited:
**EP-A- 0 412 849**

- **-: "Extended efficacy spectrum of azole pesticides." RESEARCH DISCLOSURE, 380, P802, (NO. 38028), vol. 380, December 1995 (1995-12), page 802, XP000549823**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 686 971 B1

## Description

The invention is related to the use of haloarylpyrazole compounds for deterring ticks.

Repellents and deterrents have In general the task of deterring harmful or troublesome arthropods from contacting, stinging or feeding on areas that are attractive to them, such as the skin of animals. Because of this effect compounds with repellent activity can be used to prevent diseases caused or transmitted by such arthropods. Arthropods are in particular insects, mites and ticks.

Ticks belong to the order *Acarina.* The *Ixodidae* and Argasidae species of ticks are of most economic importance and include the important species *Boophilus* spp., *Rhipicephalus* spp, *Ixodes* spp, *Hyalomma* spp., *Amblyomma* spp., *Haemaphysalis* spp., *Dermacentor* spp., *Anocentor spp., Rhipicentor spp., Margaropus* (Ixodidae) and *Argas spp., Otobius spp.* and *Ornithodoros* spp. (Aragasidae), which preferably infest warm-blooded animals including livestock, such as cattle, pigs, sheep and goats, poultry such as chickens, turkeys and geese, fur-bearing animals such as mink, foxes, chinchillas, rabbits and the like, as well as companion animals such as cats, dogs and horses, but also humans.

Acarid infestation, i.e. infestation by ticks can cause significant economic loss in livestock due to poor quality hide, wool or sheep skin, poor quality meat /tissue, reduced weight gain, reduced milk production and even death as a result of the animal carrying ticks.

Ticks are responsible world-wide for the transmission and spread of many human and animal diseases. They are carriers of bacterial (e.g. rickettsial), viral and protozoal diseases and cause tick-paralysis and tick-toxicosis. Even a single tick can cause paralysis whereby its saliva penetrates into the host animal during ingestion.

Diseases caused by ticks are usually transmitted by ticks, which infest several host animals. Such diseases, for example babesiosis, anaplasmosis and theileriosis, are responsible for the death or impairment of a large number of domestic and farm animals in the entire world. In many countries of temperate climate, ixodide ticks transmit the agent of the chronically harmful Lyme's disease from wild animals to humans.

Although the harmful effects of a tick infestation on animals have been known for years, and enormous progress has been made using tick-control programmes, until now no completely satisfactory methods of controlling or eliminating these parasites have been found, and in addition, ticks have often developed resistance to chemical active ingredients.

Thus, there continues to be a need for control programs against acarid parasites, especially ticks which afflict companion animals and livestock and which are effective at low application rates, selective in biologic action and have low toxicity.

In EP 412849 certain aryl-1,2,3 triazoles and arylpyrazoles are disclosed in which the imidazol(in)e group is attached directly or indirectly through its 2-position to the triazole or pyrazole ring that have pesticidal activity. For certain of the compounds that are disclosed in European Patent No. EP 412849, systemic activity against ectoparasitic insects after oral application to an animal is described.

The efficacy of certain haloarylpyrazoles in the control of ticks (Boophilus and multi-host tick species) at a dosage of 7.5 to 15 mg/ kg in host animals has been disclosed in Research disclosure 380, P802 (No 38028) "Extended Efficacy Spectrum of Azole Pesticides" Research disclosure 380, P802 (No 38028).

The current invention proposes to improve the processes for controlling acarid parasites, especially ticks in animals, especially in companion animals, in particular in dogs.

The invention thus relates in a first aspect to the use of haloarylpyrazole compounds of formula (I)

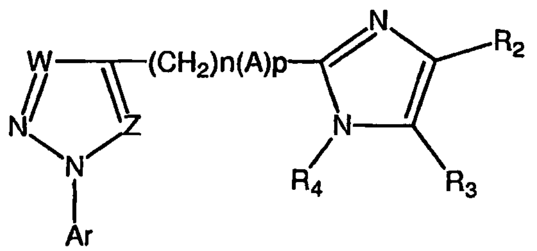

(I)

wherein

Ar is 2,6-dichloro-4- trifluoromethylphenyl or 2-nitro-4-trifluoromethylphenyl;
A is $S(O)_m$, -CH=CH-, O or NH;
W is N and Z is $CR^5$; or W is $CR^1$ and Z is N or $CF^5$;
$R^1$ is hydrogen, optionally substituted alkyl, halogen or $R^{20}S(O)_q$;
$R^2$ and $R^3$ are hydrogen, alkyl, alkenyl or alkynyl, each of which is optionally substituted, aryl, cyano, halogen, nitro, $YR^{20}$, $S(O)_2NR^8R^9$, CHO, $NR^8F^9$ or $CYNR^8R^9$;
$R^4$ is hydrogen, optionally substituted alkyl, optionally substituted alkenyl, acyl or optionally substituted alkoxycar-

bonyl;
$R^5$ is hydrogen, alkyl, optionally substituted amino or halogen;
$R^8$ and $R^9$ are the same or different and are hydrogen, optionally substituted alkyl, acyl or aryl;
$R^{20}$ is optionally substituted alkyl;
Y is O or S;
m is 0, 1 or 2;
p is 0 or 1;
n is 0, 1 our 2; and
q is 0, 1 or 2,
and in which a) any alkyl, alkoxy and alkylthio groups is of 1 to 4 carbon atoms; b) any alkenyl or alkynyl groups is of 2 to 5 carbon atoms; c) any substituted alkyl, alkoxy, alkylthio, alkenyl or alkynyl group is substituted by one or more of the same or different groups selected from Halogen, $YR^{20}$, dihalocyclopropyl, cyano, nitro, optionally substituted amino, acyloxy and aryl; d) any aryl group is phenyl, optionally substituted, by halogen, alkyl, haloalkyl, alkoxy, haloalkoxy, alkylthio, haloalkylthio, haloalkylsulphonyl, cyano or nitro; e) any acyl group is alkanoyl of 1 to 4 carbon atoms, or alkylsulphonyl or haloalkylsulphonyl; and f) any optionally substituted amino groups is of formula $NR^8R^9$, with the proviso that when W is $CR^1$ and Z is $CR^5$ and n and p are both 0, $R^4$ is not alkyl, for the manufacturing of a medicament for deterring ticks in animals.

Haloarylpyrazole compounds of formula (I) have been described in EP 0412849. Methods for the preparation of these compounds are also disclosed in EP 0412849.

A prominent representative of an haloarylpyrazole compound of formula (I) to be used in the non-therapeutical process for deterring ticks is 5-chloro-1-(2,6-dichloro-4-trifluoromethylphenyl)-4-(4,5-dicyano-1H-imidazol-2-yl)-3-methyl-1H-pyrazole of formula (II):

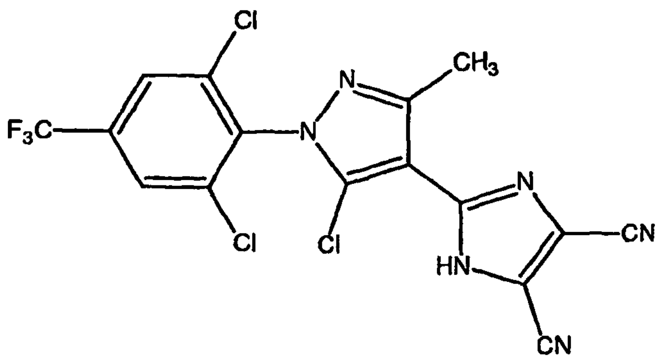
(II)

This compound is indicated as compound 22c in European Patent No. EP 0412849. The synthesis of this compound is disclosed in Example 22b of EP 0412849. In the remainder of this application this compound will be referred to as "compound 22c".

Repellents and deterrents have the task of deterring harmful or troublesome arthropods from contacting, stinging or feeding on areas that are attractive to them, such as the skin of animals. It is the object of the current invention that this repellent effect can be reached by means of prior administration of the arylpyrazole compound to the host animals. The deterrent effect after administration of the compound results in anti-attachment and anti-feeding of the parasites, especially ticks. The deterrent (repellent) effect according to the current invention is shown in the example. A significant higher number of live, non-engorged ticks were found in the pens of dogs treated with a compound according to the invention compared to the environment of the control group.

The benefit of deterring ticks is that compounds are very effective against target parasites and at the same time of low toxicity for the warm-blooded host animals. This is because their activity is based not only on the death of the target parasite, but on the parrying defence thereof (as a repellent or as a deterrent), before it attaches, bites feeds or in any other way harms the host organism. The presence of the deterring (repellent) compounds appears to disturb the parasites in such a way that they suddenly leave the treated animal without attaching or feeding or even do not infest a treated host animal at all.

In an alternative embodiment the compounds of formula (I) can be used in a non-therapeutic process for deterring ticks from animals.

Preferably the compounds of formula (I) are administered systemically. Systemic administration is the administration of the composition to an animal at a site remote from the site where the parasite resides, so that the active compound gets in contact with the parasite along with the blood, body fluids or tissue such as e.g. skin of the host animal,

and can then exhibit activity against the parasite. Systemic administration is e.g. orally, by injection, implant or by other means, for example trans-dermally by spot-on or pour-on, so that there is sufficient of the agent in the tissues or body fluids to kill the parasite feeding on the animal in the case of control or to deter the parasite.

The compound is preferably administered in an oral formulation. The term "oral formulation" means that active ingredients are formulated into a product or formulation suitable for administering to the animal via the mouth. These products or formulations include, but are not limited to tablets, capsules, liquids, gels, pastes, oral sprays, buccal formulations, powders, granules, chewable treats or animal feeds containing the active ingredient. Such formulations include pharmaceutically acceptable auxiliaries.

Pharmaceutically acceptable auxiliaries are known in the art and described in, for example, standard textbooks such as Remington: The science and Practice of Pharmacy, 20th Edition (2000), Chapter 45. Solid oral formulations, e.g. conventional tablets generally comprise the active ingredients and a diluent to assist in increasing the powder mass to a convenient size and improve compressibility, a binder to hold the compressed powder together and a lubricant to assist in densification and ejection from the tablet die. They may also contain a disintegrate, to improve disintegration and dissolution as well as stabilizers, colours and flavours. Tablets are often coated to improve appearance or taste or to alter the dissolution properties. Tablets can be designed to dissolve fast or slow, and depending on the actual volume and compressibility of the drug, large or small. They can be made chewable or to dissolve under the tongue or in the pouch of the cheek.

Conventional liquid formulations are usually solutions, suspension or emulsions of the active ingredient together with suitable diluents, solvents, flavours and colours to form a palatable dosage form.

It has been found that compound 22c - 5-chloro-1-(2,6-dichloro-4-trifluoromethylphenyl)-4-(4,5-dicyano-1H-imidazol-2-yl)-3-methyl-1-H pyrazole of formula (II), is effective against ticks when systemically applied in a regimen that employs the administration of a initial dosage of 4 mg/kg bodyweight followed by weekly administrations of 2 mg/kg for improved control of ticks. This administration scheme was proven to be effective to control tick infestation of animals and is safe to the host animal. The initial dose of 4 mg/kg can also be split into two doses of 2 mg/kg given on two consecutive days.

The compound 22c can be administered in such a regimen and as repellent to all species of animals that have tick infestation. The recipient of the composition may be a livestock animal e.g. sheep, cattle, pig, goat, a laboratory test animal, e.g. guinea pig, rat or mouse or, in particular, a companion animal e.g. dog, cat, rabbit, ferret or horse. Preferably the animal is a companion animal, more preferably a dog or cat. Especially preferred are dogs.

In general, the composition according to the invention will contain an effective amount of the active ingredients, meaning a non-toxic but sufficient amount to provide the desired control or deterring effect. Such an amount will depend on the age, condition, weight and type of the target animal.

EXAMPLE:

<u>Tick- efficacy of compound 22c.</u>

Two groups of 6 dogs each were infested with ticks *(Rhipicephalus sanguineus)* before and at different time points after treatment. Group I was treated orally with Compound 22c at a dose of 4 mg/kg bodyweight (bw) followed by 3 weekly doses of 2 mg/kg bw. Group II remained untreated as positive control.
The parasite burden of individual dogs was assessed 48 h, 72 h or 120 h after infestation by removing and counting of ticks. Ticks were classed according to vitality (dead/alive) and the parasitic status of ticks (engorged/unfed; attached/free).

MATERIALS AND METHODS

Study animals

| | |
|---|---|
| Species: | Domestic dog |
| Number: | 12 |

Health status: The dogs were healthy at the start of the study as determined by a veterinarian on Day -2. None of the dogs had been treated with an acaricide/insecticide for at least 8 weeks prior to the study.

Animal Housing

Study animals were kept indoors in individual pens with concrete floors and a special resting area allowing the collection of parasites from the environment. The study was carried out according to the existing guidelines for the "Testing and evaluation of the efficacy of antiparasitic substances for the treatment and prevention of tick and flea infestation in dogs and cats" (EMEA/CVMP/005/00) providing maximum comfort to the dogs under study conditions.

Parasite infestation

Dogs were experimentally infested with laboratory strains of *Rhipicephalus sanguineus* (50-100 unfed adults; sex ratio 1:1) as reflected in table 1. Ticks were directly applied onto the back of the animal. For the time of parasite distribution over the host the dogs were kept quiet by sedation.
Ticks of *Rhipicephalus sanguineus* were infested on day -2, day +5, day +12, day +16, and day +23.

Product specifications

| | |
|---|---|
| Investigational Product | Compound 22c Tablet |
| Active Ingredient: | Compound 22c 10.0 % (w/w) |
| Dosage : | 4 mg/kg bodyweight -initial dose; 2 mg/kg bodyweight - maintenance dose, Application Per os |

Treatment

Treatments followed the schedule as set out below:

| Day | Number of dogs | Investigational veterinary product | Dose | Application |
|---|---|---|---|---|
| 0 | 6 | Compound 22c | 4 mg/kg bw | Per os |
| +7 | 6 | Compound 22c | 2 mg/kg bw | Per os |
| +14 | 6 | Compound 22c | 2 mg/kg bw | Per os |
| +21 | 6 | Compound 22c | 2 mg/kg bw | Per os |

Route and Method of Administration

The investigational product was applied directly to the back of the tongue to induce swallowing. To calculate the doses body weights were used. The smallest unit that had been used was half a tablet. Tablets were not scored but were broken into even pieces. Where necessary the dose was rounded to the nearest half tablet. The administered doses were recorded.

Assessments:

Evaluation of Tick Numbers

**[0033]** Each assessment started with the dogs of the treatment group to prevent cross-contamination of treated dogs with ticks of the control group.
Tick assessments on the dogs were made on the day +2, day +7, day +14, day +21 and day +26. Tick counts were made 48 hours after the first treatment and 48 hours, 72 hours or 120 hours after re-infestation. For counting all ticks were removed from the animals and grouped into the following categories:

| Category | General findings | Attachment status | Effect |
|---|---|---|---|
| 1 | live | Free | no |
| 2 | live | attached - unengorged | no (except single ticks) |

(continued)

| Category | General findings | Attachment status | Effect |
|---|---|---|---|
| 3 | live | attached - engorged | no (except single ticks) |
| 4 | killed | Free | yes |
| 5 | killed | attached - unengorged | yes |
| 6 | killed | attached - engorged | no (except single ticks) |

Evaluation of Repelled Ticks

To assess the number of repelled ticks due to treatment with compound 22c, live, unattached ticks were collected daily from the pens of the dogs after each infestation. The total number of collected ticks were determined on each assessment-day to determine the number of unattached (control dogs) and repelled ticks (treated dogs) for each group.

Calculation of Efficacy

Efficacy calculation was based on the arithmetic means of number of ticks on treated dogs compared to the control group. For calculation of efficacy (%), the following formula (according to Abbot's formula) is used:

$$\text{Efficacy} = 100 \times (m_c - m_r)/m_c$$

| | |
|---|---|
| Control group ($m_c$): | mean number of live ticks on the host animals |
| Treatment group ($m_r$): | mean number of live (category 1-3) or killed, engorged ticks (category 6) on the host animal |

The number of life, unattached ticks collected from the pens of both groups were statistically compared using the Pearson asymptotic and exact Chi-Square-Test to determine a repellent / deterrent effect. If the statistical evaluation showed significance for repellence, the free ticks collected from the pens were not considered for calculation of efficacy.

RESULTS

The overall efficacy against ticks (Rhipicephalus sanguineus) is given in Figure 1. The repellence effect is given in Figure 2.

EFFICACY AGAINST *RHIPIPCEPHALUS SANGUINEUS*

Day +2

Each dog was infested with 50 *Rhipicephalus sanguineus* on day -2. On day 0 the initial dose of 4 mg/kg bw was applied to each dog of the treatment group. Ticks were assessed 48 hours after treatment and recorded.
44 live, non-engorged ticks were found in the pens of treated dogs whereas not a single live, free tick was found in the environment of the control group. Statistics strongly suggest a repellent effect of compound 22c (p <0.0001). Repellence is a parameter of control. The overall efficacy was 93.8 %.

Day+7:

Each dog was infested with 100 *Rhipicephalus sanguineus* on day +5 after treatment. 48 hours after infestation, the assessment was conducted as described.
As on day +2 after treatment, there was a significant difference of free, live ticks in the pens of treated dogs (n = 91) versus free, live ticks collected from control pens (n = 11). Repellence was highly significant (p <0.0001). The overall efficacy was 93.2 %.

Day +14:

Each dog was infested with 80 *Rhipicephalus sanguineus* on day +12 after the last treatment (d +7). 48 hours after infestation the assessment was conducted.
As before, there was a significant difference ($p < 0.0001$) of free, live ticks in the pens of treated dogs ($n = 60$) versus free, live ticks collected from control pens ($n = 17$) confirming the repellent hypothesis. All 60 live ticks collected from the pens of the treated group were visibly damaged indicating the ticks had been in contact with the drug but had not firmly attached but left the host. The overall efficacy was 94.6 %.

Day +21

Each dog was infested with 60 *Rhipicephalus sanguineus* on day +16; 48 hours after the last treatment (d +14). The tick assessment was conducted 120 hours after infestation (day +21). The time of assessment was extended to investigate if the efficacy increased when ticks were exposed to the drug for longer than 48 hours. As before, there was a significant difference ($p < 0.0001$) of free, live ticks in the pens of treated dogs ($n = 60$) versus free, live ticks collected from control pens ($n = 20$) confirming the repellent hypothesis. 59 of the 60 live, free ticks of the treated group were visibly damaged indicating exposure to the drug without attaching firmly to the host. The overall efficacy was 99.4 %.

Day +26

Each dog was infested with 60 *Rhipicephalus sanguineus* on day +23, 48 hours after the last treatment (d +21). The assessment was conducted 72 hours after the infestation.
34 of the 35 live, free ticks collected from pens of the treated group were visibly damaged indicating that they had been efficiently repelled from the host ($p < 0.0001$). The overall efficacy was 98.3 %.

Overall, the Compound 22c showed prophylactic activity against ticks preventing attachment and feeding (repellence). Significant numbers of live but visibly damaged ticks were collected from the pens of treated dogs after each infestation indicating ticks failed to firmly attach to the host. Under natural challenge conditions this will be seen as a preventive effect against ticks.

Against *Rhipicephalus sanguineus* initial efficacy of 94 % was reached within 48 hours after the first treatment. Challenge infestations 48 hours after dosing (day 16; day 23) were controlled in the range of 98 - 99 %. Ticks infested 120 hours after the dosing (day 5; day 12) were controlled with 93 - 95 %. For *Rhipicephalus sanguineus,* therapeutic tick control was achieved throughout the study (28 days) with the weekly dosing schedule. In conclusion, Compound 22c was highly efficacious against *Rhipicephalus sanguineus.*

Overall, Compound 22c applied at a weekly dose of 2 mg/kg bw after an initial dosage of 4 mg/kg bw was well tolerated and demonstrated an exceptional acaricidal potential as well as the potential to prevent attachment and feeding of ticks.

## Claims

1. Use of haloarylpyrazole compounds of formula (I)

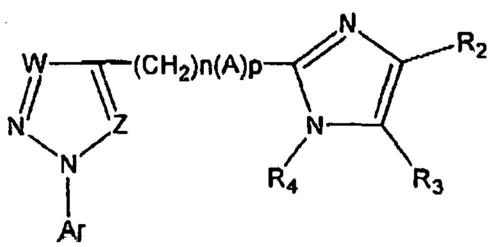

(I)

wherein

Ar is 2,6-dichloro-4- trifluoromethylphenyl or 2-nitro-4-trifluoromethylphenyl;
A is $S(O)_m$, -CH=CH-, O or NH;
W is N and Z is $CR^s$; or W is $CR^1$ and Z is N or $CR^5$;
$R^1$ is hydrogen, optionally substituted alkyl, halogen or $R^{20}S(O)_q$;
$R^2$ and $R^3$ are hydrogen, alkyl, alkenyl or alkynyl, each of which is optionally substituted, aryl, cyano, halogen,

nitro, $YR^{20}$, $S(O)_2NR^8R^9$. CHO, $NR^8R^9$ or $CYNR^8R^9$;
$R^4$ is hydrogen, optionally substituted alkyl, optionally substituted alkenyl, acyl or optionally substituted alkoxycarbonyl;
$R^5$ is hydrogen, alkyl, optionally substituted amino or halogen;
$R^8$ and $R^9$ are the same or different and are hydrogen, optionally substituted alkyl, acyl or aryl;
$R^{20}$ is optionally substituted alkyl;
Y is O or S;
m is 0, 1 or 2;
p is 0 or 1;
n is 0, 1 or 2; and
q is 0. 1 or 2,
and in which a) any alkyl, alkoxy and alkylthio groups is of 1 to 4 carbon atoms; b) any alkenyl or alkynyl groups is of 2 to 5 carbon atoms; c) any substituted alkyl, alkoxy, alkylthio, alkenyl or alkynyl group is substituted by one or more of the same or different groups selected from halogen, $YR^{20}$, dihalocyclopropyl, cyano, nitro, optionally substituted amino, acyloxy and aryl; d) any aryl group is phenyl, optionally substituted, by halogen, alkyl, haloalkyl, alkoxy, haloalkoxy, alkylthio, haloalkylthio, haloalkylsulphonyl, cyano or nitro; e) any acyl group is alkanoyl of 1 to 4 carbon atoms, or alkylsulphonyl or haloalkylsulphonyl; and f) any optionally substituted amino groups is of formula $NR^8R^9$, with the proviso that when W is $CR^1$ and Z is $CR^5$ and n and p are both 0, $R^4$ is not alkyl, for the manufacture of a medicament for deterring ticks in animals.

2. Use according to claim 1 **characterised in that** the compound is 5-chloro-1-(2, 6-dichloro-4-trifluoromethylphenyl)-4-(4,5-dicyano-1H-imidazol-2-yl)-3-methyl-1-H pyrazole.

3. Use according to claims 1 or 2, **characterised in that** the compound is applied systemically to an animal.

4. Use according to claim 3, **characterised in that** the compound is applied orally to an animal.

5. Use according to claim 1 to 4, **characterised in that** the compound is applied as a tablet to an animal.

6. Use according to claims 1 to 5 **characterised in that** the compound is applied to a dog or cat.

7. Use according to claims 1 to 6, **characterised in that** the compound is applied in an initial dose of 4 mg / kg bodyweight of the animal followed by weekly administration of doses of 2 mg/kg bodyweight of the animal.

8. Use of 5-chloro-1-(2,6-dichloro-4-trifluoromethylphenyl)-4-(4,5-dicyano-1H-imidazol-2-yl)-3-methyl-1-H pyrazole for the manufacturing of a medicament for deterring ticks by oral administration to animals in an initial dose of 4 mg / kg bodyweight of the animal followed by weekly administration of doses of 2 mg/kg bodyweight of the animal.

9. Use according to claim 8, **characterised in that** 5-chloro-1-(2,6-dichloro-4-trifluoromethylphenyl)-4-(4,5-dicyano-1H-imidazol-2-yl)-3-methyl-1-H pyrazole is administered as a tablet.

10. Use according to claim 8 or 9, **characterised in that** 5-chloro-1-(2,6-dichloro-4-trifluoromethylphenyl)-4-(4,5-dicyano-1H-imidazol-2-yl)-3-methyl-1-H pyrazole is administered to a dog.

**Patentansprüche**

1. Verwendung von Halogenarylpyrazolverbindungen der Formel (I)

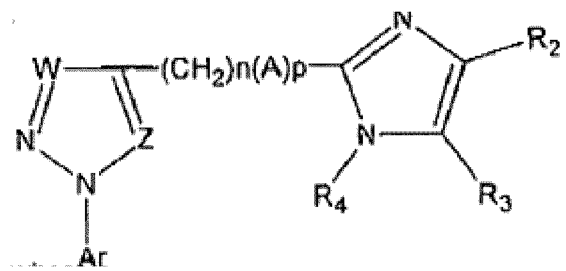

(I)

worin

Ar 2,6-Dichlor-4-trifluormethylphenyl oder 2-Nitro-4-trifluormethylphenyl bedeutet;
A $S(O)_m$, -CH=CH-, 0 oder NH bedeutet;
W N bedeutet und Z $CR^5$ bedeutet; oder W $CR^1$ bedeutet und Z N oder $CR^5$ bedeutet;
$R^1$ Wasserstoff, gegebenenfalls substituiertes Alkyl, Halogen oder $R^{20}S(O)_q$ bedeutet;
$R^2$ und $R^3$ Wasserstoff, Alkyl, Alkenyl oder Alkinyl, die jeweils gegebenenfalls substituiert sind, Aryl, Cyano, Halogen, Nitro, $YR^{20}$, $S(O)_2NR^8R^9$, CHO, $NR^8R^9$ oder $CYNR^8R^9$ bedeuten;
$R^4$ Wasserstoff, gegebenenfalls substituiertes Alkyl, gegebenenfalls substituiertes Alkenyl, Acyl oder gegebenenfalls substituiertes Alkoxycarbonyl bedeutet; $R^5$ Wasserstoff, Alkyl, gegebenenfalls substituiertes Amino oder Halogen bedeutet;
$R^8$ und $R^9$ gleich oder verschieden sind und Wasserstoff, gegebenenfalls substituiertes Alkyl, Acyl oder Aryl bedeuten;
$R^{20}$ gegebenenfalls substituiertes Alkyl bedeutet;
Y 0 oder S bedeutet;
m 0, 1 oder 2 bedeutet;
p 0 oder 1 bedeutet;
n 0, 1 oder 2 bedeutet; und
q 0, 1 oder 2 bedeutet,
und worin a) jegliche Alkyl-, Alkoxy- und Alkylthiogruppe 1 bis 4 Kohlenstoffatome aufweist; b) jegliche Alkenyl- oder Alkinylgruppe 2 bis 5 Kohlenstoffatome aufweist; c) jegliche substituierte Alkyl-, Alkoxy-, Alkylthio-, Alkenyl- oder Alkinylgruppe durch eine oder mehr gleiche oder verschiedene Gruppen, ausgewählt aus Halogen, $YR^{20}$ Dihalogencyclopropyl, Cyano, Nitro, gegebenenfalls substituiertem Amino, Acyloxy und Aryl, substituiert ist; d) jegliche Arylgruppe Phenyl, das gegebenenfalls durch Halogen, Alkyl, Halogenalkyl, Alkoxy, Halogenalkoxy, Alkylthio, Halogenalkylthio, Halogenalkylsulfonyl, Cyano oder Nitro substituiert ist, bedeutet; e) jegliche Acylgruppe Alkanoyl, das 1 bis 4 Kohlenstoffatome, oder Alkylsulfonyl oder Halogenalkylsulfonyl bedeutet; und f) jegliche gegebenenfalls substituierte Aminogruppe der Formel $NR^8R^9$ entspricht, mit der Maßgabe, dass, wenn W $CR^1$ bedeutet und Z $CR^5$ bedeutet und n und p beide 0 bedeuten, dann $R^4$ nicht Alkyl bedeutet,
für die Herstellung eines Arzneimittels zum Abwehren von Zecken bei Tieren.

2. Verwendung nach Anspruch 1, **dadurch gekennzeichnet, dass** es sich bei der Verbindung um 5-Chlor-1-(2,6-dichlor-4-trifluormethylphenyl)-4-(4,5-dicyano-1H-imidazol-2-yl)-3-methyl-1H-pyrazol handelt.

3. Verwendung nach Anspruch 1 oder 2, **dadurch gekennzeichnet, dass** die Verbindung einem Tier systemisch verabreicht wird.

4. Verwendung nach Anspruch 3, **dadurch gekennzeichnet, dass** die Verbindung einem Tier oral verabreicht wird.

5. Verwendung nach den Ansprüchen 1 bis 4, **dadurch gekennzeichnet, dass** die Verbindung einem Tier in Tablettenform verabreicht wird.

6. Verwendung nach den Ansprüchen 1 bis 5, **dadurch gekennzeichnet, dass** die Verbindung an einen Hund oder eine Katze verabreicht wird.

7. Verwendung nach den Ansprüchen 1 bis 6, **dadurch gekennzeichnet, dass** die Verbindung mit einer Anfangsdosis von 4 mg/kg Körpergewicht des Tiers und anschließend durch eine wöchentliche Verabreichung von Dosen von 2 mg/kg Körpergewicht des Tiers verabreicht wird.

8. Verwendung von 5-Chlor-1-(2,6-dichlor-4-trifluormethylphenyl)-4-(4,5-dicyano-1H-imidazol-2-yl)-3-methyl-1H-pyrazol für die Herstellung eines Arzneimittels zum Abwehren von Zecken durch orale Verabreichung an Tiere mit einer Anfangsdosis von 4 mg/kg Körpergewicht des Tiers und anschließend durch eine wöchentliche Verabreichung von Dosen von 2 mg/kg Körpergewicht des Tiers.

9. Verwendung nach Anspruch 8, **dadurch gekennzeichnet, dass** 5-Chlor-1-(2,6-dichlor-4-trifluormethylphenyl)-4-(4,5-dicyano-1H-imidazol-2-yl)-3-methyl-1H-pyrazol in Tablettenform verabreicht wird.

10. Verwendung nach Anspruch 8 oder 9, **dadurch gekennzeichnet, dass** 5-Chlor-1-(2,6-dichlor-4-trifluormethylphenyl)-4-(4,5-dicyano-1H-imidazol-2-yl)-3-methyl-1H-pyrazol an einen Hund verabreicht wird.

## Revendications

**1.** Utilisation de composés d'haloarylpyrazole de formule (I)

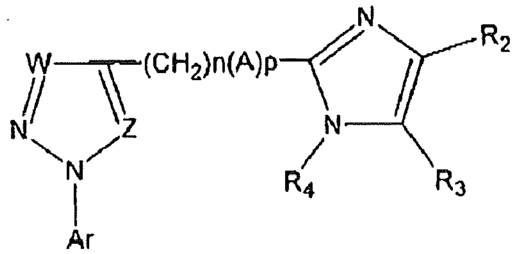

(I)

dans laquelle

Ar est 2,6-dichloro-4-trifluorométhylphényle ou 2-nitro-4-trifluorométhylphényle ;
A est $S(O)_m$, -CH=CH-, 0 ou NH ;
W est N et Z est $CR^6$ ou W est $CR^1$ et Z est N ou $CR^5$ ;
$R^1$ est hydrogène, alkyle éventuellement substitué, halogène ou $R^{20}S(O)_q$ ;
$R^2$ et $R^3$ sont hydrogène, alkyle, alcényle ou alcynyle, dont chacun est éventuellement substitué, aryle, cyano, halogène, nitro, $YR^{20}$, $S(O)_2NR^8R^9$, CHO, $NR^8R^9$ ou $CYNR^8R^9$ ; $R^4$ est hydrogène, alkyle éventuellement substitué, alcényle éventuellement substitué, acyle ou alcoxycarbonyle éventuellement substitué ;
$R^5$ est hydrogène, alkyle, amino éventuellement substitué ou halogène ;
$R^8$ et $R^9$ sont identiques ou différents et sont hydrogène, alkyle éventuellement substitués, acyle ou aryle ;
$R^{20}$ est alkyle éventuellement substitué ;
Y est 0 ou S ;
m est 0, 1 ou 2 ;
p est 0 ou 1 ;
n est 0, 1 ou 2 ; et
q est 0, 1 ou 2,
et dans laquelle a) tout groupement alkyle, alcoxy et alkylthio est de 1 à 4 atomes de carbone ; b) tout groupement alcényle ou alcynyle est de 2 à 5 atomes de carbone ; c) tout groupement alkyle, alcoxy, alkylthio, alcényle ou alcynyle substitué est substitué par un ou plusieurs groupements identiques ou différents choisis parmi halogène, $YR^{20}$, dihalogénocyclopropyle, cyano, nitro, amino éventuellement substitué, acyloxy et aryle; d) tout groupement aryle est phényle, éventuellement substitué par halogène, alkyle, halogénoalkyle, alcoxy, halogénoalcoxy, alkylthio, halogénoalkylthio, halogénoalkylsulfonyle, cyano ou nitro ; e) tout groupement acyle est alcanoyle de 1 à 4 atomes de carbone ou alkylsulfonyle ou halogénoalkylsulfonyle ; et f) tout groupement amino éventuellement substitué est de formule $NR^8R^9$, à condition que lorsque W est $CR^1$ et Z est $CR^5$ et n et p sont tous deux 0, $R^4$ ne soit pas alkyle, pour la fabrication d'un médicament destiné à empêcher des tiques chez les animaux.

**2.** Utilisation selon la revendication 1, **caractérisée en ce que** le composé est le 5-chloro-1-(2,6-dichloro-4-trifluorométhylphényl)-4-(4,5-dicyano-1H-imidazol-2-yl)-3-méthyl-1H-pyrazole.

**3.** Utilisation selon la revendication 1 ou 2, **caractérisée en ce que** le composé est appliqué par voie systémique sur un animal.

**4.** Utilisation selon la revendication 3, **caractérisée en ce que** le composé est administré par voie orale à un animal.

**5.** Utilisation selon les revendications 1 à 4, **caractérisée en ce que** le composé est administré sous forme de comprimé à un animal.

**6.** Utilisation selon les revendications 1 à 5, **caractérisée en ce que** le composé est appliqué sur un chien ou un chat.

**7.** Utilisation selon les revendications 1 à 6, **caractérisée en ce que** le composé est appliqué à une dose initiale de 4 mg/kg de poids corporel sur un animal, suivie d'une administration hebdomadaire de doses de 2 mg/kg de poids corporel de l'animal.

8. Utilisation du 5-chloro-1-(2,6-dichloro-4-trifluorométhylphényl)-4-(4,5-dicyano-1H-imidazol-2-yl)-3-méthyl-1H-pyrazole pour la fabrication d'un médicament destiné à empêcher les tiques par administration par voie orale à des animaux dans une dose initiale de 4 mg/kg de poids corporel de l'animal, suivie de l'administration hebdomadaire de doses de 2 mg/kg de poids corporel de l'animal.

9. Utilisation selon la revendication 8, **caractérisée en ce que** le 5-chloro-1-(2,6-dichloro-4-trifluorométhylphényl)-4-(4,5-dicyano-1H-imidazol-2-yl)-3-méthyl-1H-pyrazole est administré sous forme de comprimé.

10. Utilisation selon la revendication 8 ou 9, **caractérisée en ce que** le 5-chloro-1-(2,6-dichloro-4-trifluorométhylphényl)-4-(4,5-dicyano-1H-imidazol-2-yl)-3-méthyl-1H-pyrazole est administré à un chien.

FIGURE 1: Overall efficacy of compound 22c

Assessment 1 = day 2, Assessment 2 = day 7, Assessment 3= day 14, Assessment 4= day 21, Assessment 5 = day 26

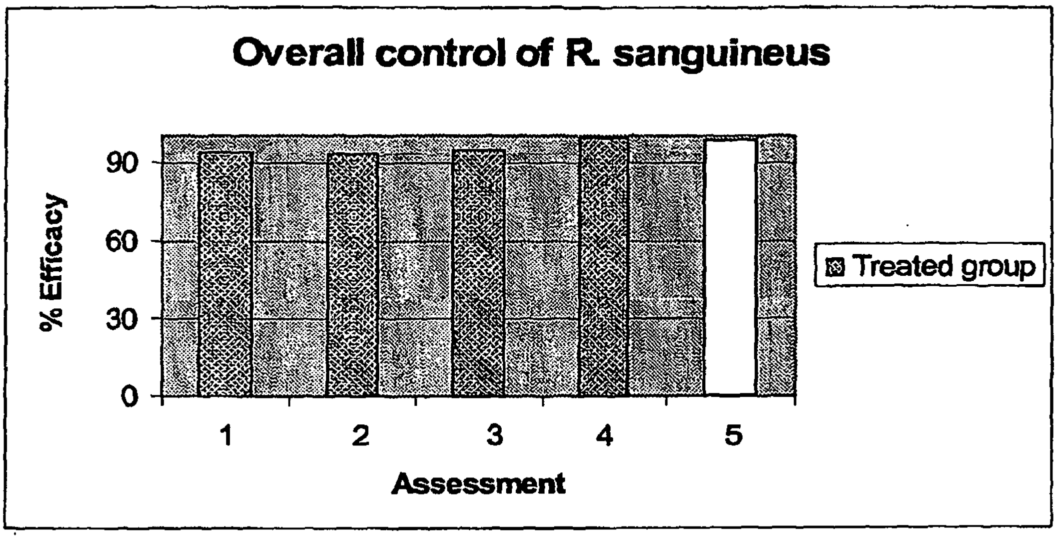


FIGURE 2: Repellent efficacy of compound 22c

Assessment 1 = day 2, Assessment 2 = day 7, Assessment 3= day 14, Assessment 4= day 21, Assessment 5 = day 26

Repelled ticks = treated group unattached ticks-= control group

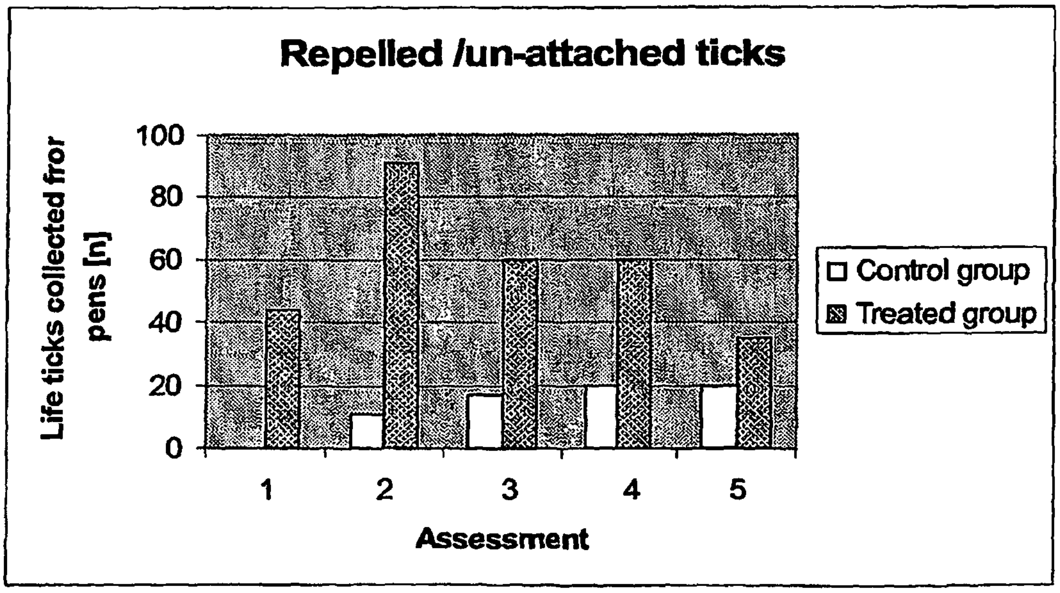

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 412849 A
- EP 0412849 A


### Non-patent literature cited in the description


- Remington: The science and Practice of Pharmacy. 2000